# EUROPÄISCHE PATENTSCHRIFT

(11) **EP 0 825 168 B1**
(45) Veröffentlichungstag und Bekanntmachung des Hinweises auf die Patenterteilung: **21.11.2001**
(21) Anmeldenummer: 97114056.1
(22) Anmeldetag: 14.08.1997
(51) Int. Cl.: C07C 47/24, C07C 43/15, C07C 41/54, C07C 41/28, C07C 45/42

(54) **Herstellung eines gamma-Halogentiglinaldehyds**
Preparation of gamma-halotiglinaldehydes
Préparation de gamma-halogénotiglinaldéhydes

(30) Priorität: 19.08.1996 EP 96113248
(43) Veröffentlichungstag der Anmeldung: 25.02.1998
(60) Teilanmeldung: 00101566.8
(73) Patentinhaber: F. HOFFMANN-LA ROCHE AG, 4070 Basel (CH)
(72) Erfinder: Burdet, Bruno, 68390 Baldersheim (FR); Rüttimann, August, 4144 Arlesheim (CH)
(74) Vertreter: Kellenberger, Marcus, Dr.

(56) Entgegenhaltungen:
- S. M. MAKIN: "The enol synthesis of polyenes" PURE AND APPLIED CHEMISTRY, Bd. 47, Nr. 2/3, 1976, Seiten 173-181, XP002055722
- S. M. MAKIN ET AL: "The chemistry of unsaturated ethers X. Addition reactions of 1-alkoxy-1,3-dienes" JOURNAL OF GENERAL CHEMISTRY OF THE USSR, Bd. 32, Nr. 4, April 1962, Seiten 1088-1092, XP002055723
- S. M. MAKIN: "Chemistry of unsaturated ethers XIV. A new method of obtaining retinene (vitamin A aldehyde)" JOURNAL OF GENERAL CHEMISTRY OF THE USSR, Bd. 32, Nr. 10, Oktober 1962, Seiten 3105-3106, XP002055724
- G. GAST ET AL: "Synthèse du nuciféral-(E)-(RS)" HELVETICA CHIMICA ACTA, Bd. 54, Nr. 5, 12.Juli 1971, Seiten 1369-1373, XP002055725
- L. CERVENY ET AL: "Catalytic splitting of acetals to unsaturated ethers" J. CHEM. TECH. BIOTECHNOL., Bd. 58, Nr. 3, 1993, Seiten 211-214, XP000404018

## Beschreibung

Die vorliegende Erfindung betrifft ein neues Verfahren zur Herstellung eines γ-Halogentiglinaldehyds. Der so hergestellte γ-Halogentiglinaldehyd kann zur Herstellung von γ-Acetoxy-tiglinaldehyd oder von gewissen C₅-Wittigaldehydhalogeniden verwendet werden. Diese Produkte sind bekannte, wichtige Zwischenprodukte für die Herstellung von verschiedenen Apocarotinalen und Diapocarotinalen sowie von Vitamin A.

Der γ-Chlortiglinaldehyd sowie die entsprechende γ-Brom-Verbindung sind bekannt und können nach verschiedenen bekannten Methoden hergestellt werden, und zwar ausgehend von Methylglyoxal-dimethylacetal [siehe Belgische Patentschrift 633.007, Deutsche Offenlegungsschriften (DOS) 1.188.577 und 1.952.649 sowie Finn. Chem. Lett. 1984, 102 ff.], von Isopren [DOS 2.620.968, US-Patentschrift 4.288.635, J. Org. Chem. 41, 1648 ff. (1976), Rec. Trav. Chim. Pays-Bas 99, 384 ff. (1980) sowie Tetrahedron 34, 2205 ff. (1978)], von 3-Methyl-2-butenylchlorid [Nippon Kagaku Kaishi 1975, 2246 ff./Chem. Abs. 84(9), 58575s], von Tiglinaldehyd [Tetr. Lett. 20, 1719 ff. (1973)] oder von 1-Methoxy/Ethoxy-2-methyl-1,3-butadien [J. Gen. Chem. USSR 32, 1088 ff. (1962; englische Uebersetzung), Russian Chem. Rev. 38, 237 ff. (1969), Pure & Appl. Chem. 47, 173 ff. (1976), J. Gen. Chem. USSR 32, 3105 ff. (1962; englische Uebersetzung) sowie Helv. Chim Acta 54, 1369 ff. (1971)]. Was die letztgenannte Methode anbelangt, so konnte mittels N-Bromsuccinimid in Methanol oder Ethanol das 1-Alkoxy-2-methyl-1,3-butadien zum entsprechenden γ-Bromtiglinaldehyd-dialkylacetal bromalkoxyliert werden, aus welchem seinerseits je nach Wunsch das entsprechende Acetal des γ-Acetoxy-tiglinaldehyds und anschliessend der γ-Acetoxy-tiglinaldehyd bzw. der γ-Bromtiglinaldehyd hergestellt werden konnte. Aus der diesbezüglichen Fachliteratur geht nicht hervor, dass andere Bromierungsmittel als N-Bromsuccinimid zur Herstellung von γ-Bromtiglinaldehyd aus dem 1-Alkoxy-2-methyl-1,3-butadien eingesetzt worden sind. Ferner ist auch die Herstellung von γ-Chlortiglinaldehyd ausgehend von diesem 1,3-Butadienderivat durch Haloalkoxylierung aus der Fachliteratur nicht bekannt. Man kann allerdings erwarten, dass anstelle von N-Bromsuccinimid die entsprechende N-Chlor-Verbindung, d.h. N-Chlorsuccinimid, zu diesem Zweck und auf analoge Weise verwendet werden könnte.

Es wurde nun gefunden, dass man ein 1-Alkoxy-2-methyl-1,3-butadien auch direkt mittels eines zu diesem Zweck bisher noch nie verwendeten Halogenierungsmittels in Gegenwart eines Alkohols unter Bildung des diesbezüglichen γ-Halogentiglinaldehyd-dialkylacetals haloalkoxylieren und nach anschliessender Hydrolyse dieses Acetals den γ-Halogentiglinaldehyd erhalten kann. Bei dem Halogenierungsmittel handelt es sich um ein Alkalimetall- oder Erdalkalimetallhypochlorit oder -hypobromit, das tert.-Butylhypochlorit, das N-Bromacetamid oder das 1,3-Dichlor- oder 1,3-Dibrom-5,5-dimethyl-hydantoin. Diese Halogenierungsmittel sind bekannte Verbindungen und zum grössten Teil leicht käuflich. So ist beispielsweise die wässrige, sogenannte "Javellauge" oder "Eau de Javelle (Kaliumhypochlorit) bzw. Eau de Labarraque (Natriumhypochlorit) jeweils leicht käuflich und billig, so dass deren direkte Verwendung eine wesentliche Verbesserung gegenüber den bis anhin zu diesem Zweck verwendeten Halogenierungsmitteln, vor allem N-Bromsuccinimid, darstellt. Bei der bekannten Verwendung von N-Bromsuccinimid ergibt sich als Reaktionsnebenprodukt Succinimid, welches in einem grosstechnischen Produktionsverfahren regeneriert werden müsste.

Die im erfindungsgemässen Verfahren als Substrate verwendeten 1-Alkoxy-2-methyl-1,3-butadiene sind sehr gut zugänglich, und zwar aus billigen Ausgangsmaterialien, wie dies weiter unten näher erläutert sein wird.

Das erfindungsgemässe Verfahren zur Herstellung von γ-Chlor- oder γ-Bromtiglinaldehyd der allgemeinen Formel

HalH₂C-CH=C(CH₃)-CHO I

worin Hal Chlor oder Brom bedeutet,
ist dadurch gekennzeichnet, dass man ein 1-Alkoxy-2-methyl-1,3-butadien der allgemeinen Formel

H₂C=CH-C(CH₃)=CH-OR¹ II

worin R¹ C₁₋₄-Alkyl bedeutet,
mittels eines Halogenierungsmittels, welches aus einem Alkalimetallhypochlorit, einem Alkalimetallhypobromit, einem Erdalkalimetallhypochlorit, einem Erdalkalimetallhypobromit, tert.Butylhypochlorit, N-Bromacetamid, 1,3-Dichlor-5,5-dimethyl-hydantoin und 1,3-Dibrom-5,5-dimethyl-hydantoin ausgewählt ist, in einem Alkohol R²OH, worin R² C₁₋₄-Alkyl bedeutet, haloalkoxyliert, und das so erhaltene γ-Halogentiglinaldehyd-dialkylacetal der allgemeinen Formel

HalH₂C-CH=C(CH₃)-CH(OR¹)(OR²) III

worin Hal, R¹ und R² die oben angegebenen Bedeutungen besitzen, zum gewünschten γ-Chlor- bzw. γ-Bromtiglinaldehyd der Formel I hydrolysiert.

In der obigen Definition des erfindungsgemässen Verfahrens sind unter dem Ausdruck "C₁₋₄-Alkyl" sowohl geradkettige als auch (ab C₃) verzweigte Alkylgruppen zu verstehen. Vorzugsweise ist diese Alkylgruppe allerdings Methyl oder Ethyl, was sowohl für R¹ als auch für R² gilt.

Bei dem im ersten Verfahrenschritt verwendbaren Alkalimetall- oder Erdalkalimetallhypochlorit oder -hypobromit (nachfolgend verallgemeinert als "Metallhypohalogenit" bezeichnet) handelt es sich insbesondere um das Natrium- oder Kaliumhypochlorit/-bromit bzw. um das Calciumhypochlorit/-hypobromit. Vorzugsweise wird als Chlorierungsmittel Natrium- oder Kaliumhypochlorit bzw. als Bromierungsmittel Natrium- oder Kalium-hypobromit verwendet.

Unter Verwendung der meisten Metallhypohalogenite, d.h. der Alkalimetallhypohalogenite, erfolgt die Haloalkoxylierung des ersten Verfahrensschritts zweckmässigerweise unter Einsatz einer wässrigen Lösung des Metallhypohalogenits, wobei auch der mit Wasser mischbare Alkohol R²OH im Reaktionsgemisch zwingend vorhanden ist. Als wässrige Kaliumhypochloritlösung, zum Beispiel, eignet sich insbesondere eine leicht käufliche "Javellauge", die eine Konzentration von etwa 13% w/w (Gewichtsprozent) aufweist. Wässrige Kaliumhypochloritlösungen niedrigerer oder höherer Konzentrationen als 13% w/w können jedoch ebenfalls erfolgreich verwendet werden, was auch für die entsprechende wässrige Natriumhypochlorit-, Natriumhypobromit- bzw. Kaliumhypobromitlösung zutrifft. Man verwendet zweckmässigerweise eine wässrige Lösung des Alkalimetallhypochlorits oder -hypobromits, deren Konzentration zwischen 5% w/w und 30% w/w liegt. Vorzugsweise beträgt diese Hypohalogenit-Konzentration zwischen 10% w/w und 20% w/w. Beim Einsatz der restlichen Halogenierungsmittel wird jedes zweckmässigerweise als solches, d.h. nicht in einer wässrigen Lösung, verwendet. Tert.Butylhypochlorit ist eine Flüssigkeit; Calciumhypochlorit, N-Bromacetamid, 1,3-Dichlor-5,5-dimethyl-hydantoin und 1,3-Dibrom-5,5-dimethyl-hydantoin sind hingegen Festkörper bei normalem Druck und Raumtemperatur. Ferner werden zweckmässigerweise pro Aequivalent 1-Alkoxy-2-methyl-1,3-butadien der Formel II zwischen 1,1 und 1,4 Aequivalente des ein Halogenatom aufweisenden Halogenierungsmittels oder zwischen 0,55 und 0,7 Aequivalente Erdalkalimetallhypochlorit oder -hypobromit oder 1,3-Dichlor- oder 1,3-Dibrom-5,5-dimethyl-hydantoin (zwei Halogenatome aufweisendes Halogenierungsmittel) eingesetzt. Zudem wird der Alkohol R²OH im allgemeinen in einem grossen Ueberschuss, zweckmässigerweise in einem zehn- bis dreissigfachen Ueberschuss, eingesetzt, zumal er gleichzeitig als Lösungsmittel für die Haloalkoxylierungsreaktion dient. Die Haloalkoxylierung erfolgt zweckmässigerweise im Temperaturbereich von -20°C bis zur Raumtemperatur, vorzugsweise bei Temperaturen zwischen -10°C und +5°C. Auf diese Weise wird die Haloalkoxylierung normalerweise in bis zu etwa drei Stunden Reaktionszeit beendet.

Im besonderen Fall der Verwendung von Natriumhypobromit als Bromierungsmittel wird dieses vorteilhaft vor der Haloalkoxylierung frisch hergestellt, und zwar zweckmässigerweise durch Eintropfen von Brom in wässrige Natronlauge bei etwa 0°C. Dabei verwendet man vorzugsweise etwa äquimolare Mengen Brom und Natriumhydroxid (als solches).

Die sich der Haloalkoxylierung anschliessende und als zweiten Verfahrensschritt darstellende Hydrolyse des γ-Halogentiglinaldehyd-dialkylacetals der Formel III zum gewünschten γ-Chlor- bzw. γ-Bromtiglinaldehyd der Formel I kann nach Isolierung des Acetals aus dem Reaktionsgemisch durchgeführt werden. Allerdings erweist es sich als zweckmässiger, keine derartige vollendete Isolierung und nachfolgende Hydrolyse vorzunehmen, sondern unmittelbar nach Vorliegen einer in der Aufarbeitung erhaltenen Lösung des Acetals in einem organischen Lösungsmittel dessen Hydrolyse in dieser Lösung vorzunehmen. Die Aufarbeitung und anschliessende Hydrolyse kann zweckmässigerweise erfolgen, indem man das nach beendeter Haloalkoxylierung erhaltene Gemisch mit Wasser vereinigt, geeigneterweise durch Giessen des Gemisches auf Wasser, das daraus resultierende wässrige Gemisch (in der Regel grösstenteils eine wässrige Lösung) mit einem geeigneten organischen Lösungsmittel extrahiert und die so erhaltene Lösung des Acetals im organischen Lösungsmittel mit einer wässrigen Mineralsäure versetzt. Als Lösungsmittel für die Extraktion eignet sich insbesondere ein aliphatischer, alicyclischer oder aromatischer Kohlenwasserstoff, z.B. n-Pentan oder n-Hexan, Cyclohexan bzw. Toluol; ein halogenierter aliphatischer Kohlenwasserstoff, z.B. Methylenchlorid oder Chloroform; ein aliphatischer Ether, z.B. Diethylether oder tert.Butylmethylether; oder ein aliphatischer Ester, z.B. Ethylacetat.

Vor deren Behandlung mit Säure kann die abgetrennte Extraktionslösung gegebenenfalls gewaschen werden, und zwar zweckmässigerweise mit Wasser. Man verwendet als Mineralsäure zweckmässigerweise Schwefelsäure oder Salzsäure, wobei deren Konzentration geeigneterweise im Bereich von etwa 0,5% w/w bis etwa 20% w/w liegt und die Behandlung vorzugsweise im Temperaturbereich von etwa 0°C bis zur Raumtemperatur unter Rühren erfolgt. Die Säurebehandlung dauert beispielsweise etwa 30 Minuten bis etwa 2 Stunden.

Der so hergestellte γ-Chlor- oder γ-Bromtiglinaldehyd der Formel I kann in an sich bekannter Weise vom Reaktionsgemisch isoliert und gewünschtenfalls gereinigt werden. Typischerweise wird die wässrige Phase abgetrennt und die organische Phase, in welcher sich der gewünschte γ-Chlor- oder γ-Bromtiglinaldehyd befindet, mit Wasser und/oder wässriger Natriumchlorid- und/oder Natriumbicarbonat-Lösung gewaschen, getrocknet und eingeengt. Das so isolierte und zumindest einigermassen gewaschene Rohprodukt kann dann gewünschtenfalls weitergereinigt werden, beispielsweise durch Säulenchromatographie, z.B. unter Verwendung von Kieselgel als stationärer Phase und von solchen Eluierungsmitteln wie Cyclohexan, Ethylacetat oder Gemischen davon. Sowohl der γ-Chlor- als auch der γ-Bromtiglinaldehyd sind bei normalem Druck und Raumtemperatur Flüssigkeiten. Alternativ zur Isolierung und eventueller Reinigung kann das Hydrolyseprodukt der Formel I unmittelbar in einem weiteren Verfahrensschritt, z.B. zum γ-Acetoxy-tiglinaldehyd oder zu einem entsprechenden Wittigaldehyd-chlorid oder -bromid, im Rahmen eines "Durchprozesses" umgesetzt werden. Dabei setzt man das Rohprodukt der Hydrolyse direkt in der anschliessenden Reaktion um, ohne das bei der Aufarbeitung der Hydrolyse verwendete organische Lösungsmittel abzutrennen, da das jeweilige Lösungsmittel auch im weiteren Verfahrensschritt verwendbar ist.

Die als Ausgangsmaterialien im erfindungsgemässen Verfahren verwendeten 1-Alkoxy-2-methyl-1,3-butadiene der Formel II sind zum Teil bekannte Verbindungen; die restlichen (neuen) können aus bekannten Ausgangsmaterialien nach an sich bekannten Methoden hergestellt werden.

So ist beispielsweise das 1-Ethoxy-2-methyl-1,3-butadien (der Formel II, worin R¹ Ethyl bedeutet) aus der Literatur seit langem bekannt [siehe u.a. J.A.C.S. 91, 3281 ff. (1969), Bull. Soc. Chim. Fr. 1963, 1646 ff., sowie J. Gen. Chem. USSR 29, 3649 ff. (1959)] und wurde jeweils durch zweimalige Abspaltung von Ethanol aus 1,1,3-Triethoxy-2-methyl-butan hergestellt. Das Butanderivat seinerseits kann durch eine seit langem bekannte Enoletherkondensation (siehe US-Patentschrift 2.165.962) aus den beiden gut zugänglichen Ausgangsmaterialien Acetaldehyd-diethylacetal und Ethyl-(1-propenyl)-ether hergestellt werden [siehe zudem J.A.C.S. 71, 3468 ff. (1949) sowie J. Gen. Chem. USSR 29, 3641 ff. (1959)]. Hierbei erwärmt man bei etwa 35°C etwa 2 bis 3 Aequivalente des Acetals pro Aequivalent Ethylpropenylether bis zu etwa 2 Stunden in Gegenwart von etwa 0,2 Molprozent Bortrifluoridetherat als Katalysator ohne Lösungsmittel und erhält das gewünschte Butanderivat in etwa 66%iger Ausbeute. Die anschliessende zweimalige Abspaltung von Ethanol aus dem 1,1,3-Triethoxy-2-methyl-butan kann gemäss dem einschlägigen Stand der Technik auf zwei verschiedenen Wegen realisiert werden:
(i) durch Abspaltung in der Flüssigphase, indem man das 1,1,3-Triethoxy-2-methyl-butan in Isochinolin enthaltend eine katalytische Menge p-Toluolsulfonsäure bei etwa 220°C zutropft und das in der Folge gebildete 1-Ethoxy-2-methyl-1,3-butadien abdestilliert. Die Ausbeuten sind bei dieser in Bull. Soc. Chim. Fr. 1963, 1646 ff. beschriebenen Methode allerdings mittelmässig (etwa 40-50%); oder
(ii) durch Abspaltung in der Gasphase bei 300-350°C unter Vakuum an einem sauren Katalysator, z.B. Mononatriumphosphat [J. Gen. Chem. USSR 29, 3649 ff. (1959)].

Auch das 1-Methoxy-2-methyl-1,3-butadien (der Formel II, worin R¹ Methyl bedeutet) ist aus der Literatur [Japanische Patentpublikation (Kokai) 50891/1989] bekannt. Es kann beispielsweise analog der oben beschriebenen Herstellung von 1-Ethoxy-2-methyl-1,3-butadien ausgehend von Acetaldehyd-dimethylacetal und Methyl-(1-propenyl)-ether über das 1,1,3-Trimethoxy-2-methyl-butan hergestellt werden.

Die restlichen 1-Alkoxy-2-methyl-1,3-butadiene der Formel II sind zum Teil bekannte Verbindungen und können analog den obenerwähnten Verbindungen hergestellt werden. Das nachfolgende Reaktionsschema stellt eine Zusammenfassung des oben näher erläuterten mehrstufigen Verfahrens dar, gemäss welchem alle 1-Alkoxy-2-methyl-1,3-butadiene der Formel II hergestellt werden können:

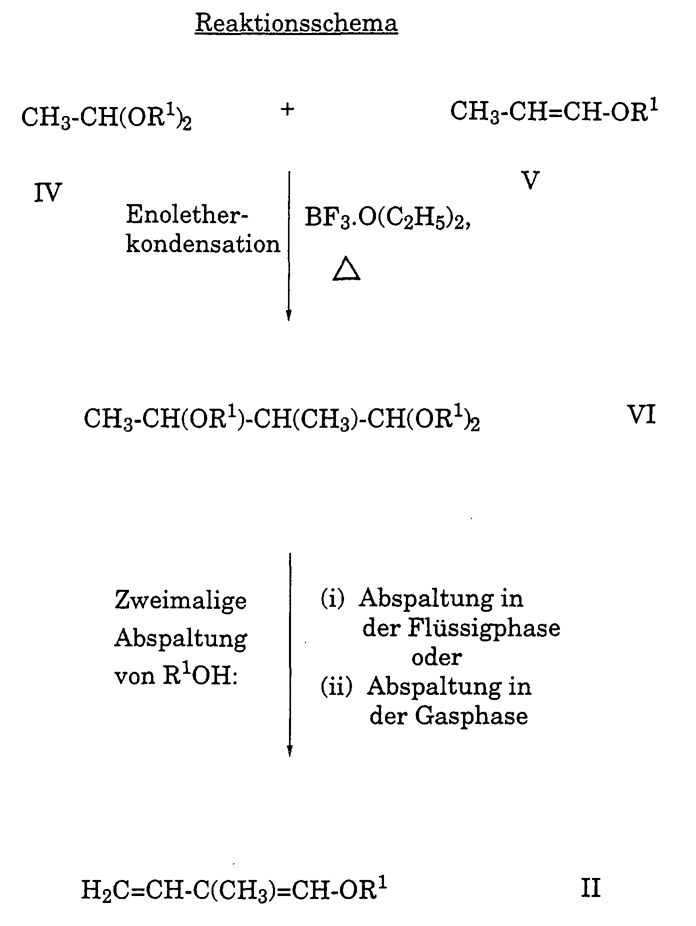

In diesem Reaktionsschema besitzt R¹ die oben angegebene Bedeutung. Das Zwischenprodukt der Formel VI und das Produkt der Formel II können auf an sich bekannte Weise isoliert und gereinigt werden.

Uebersichtsartikel zur Herstellung der 1-Alkoxy-2-methyl-1,3-butadiene befinden sich in Russian Chem. Rev. 38, 237 ff. (1969) und in Pure & Appl. Chem. 47, 173 ff. (1976); für weitere Literatur über deren Herstellung durch Gasphasenkatalyse wird auf Lieb. Ann. Chem. 568, 1 ff. (1950), Can. J. Res. B 28, 689 ff. (1950), ibid. B 25, 118 ff. (1947) sowie Chem. Ber. 77, 108 ff. (1944) verwiesen.

Die Abspaltung des Alkohols R¹OH (Dealkoxylierung) von den Trialkoxybutanen der Formel VI in der Gasphase [Methode (ii)] bei erhöhter Temperatur an einem fixierten, festen Katalysator ("Festbettkatalysator") ist bei einem technischen Prozess viel attraktiver als eine Abspaltung in der Flüssigphase [Methode (i)], da die Methode (ii) u.a. kein Lösungsmittel benötigt und eine einfache Reaktionsführung und Aufarbeitung aufweist. Daher ist die in der Gasphase erfolgende Dealkoxylierung bevorzugt. Man kann im allgemeinen als Katalysatoren ähnliche verwenden, die zur Dealkoxylierung von unsubstituierten Acetalen eingesetzt werden. Zudem wird eine derartige Dealkoxylierung zweckmässigerweise bei Temperaturen zwischen etwa 300°C und etwa 350°C durchgeführt.

Es wurde bei der Untersuchung der Dealkoxylierung nun gefunden, dass sich ein bisher zu diesem Zweck noch nie verwendeter Typ von Katalysator besonders gut eignet. Bei diesem Katalysator handelt es sich um schwach saures Aluminiumsilikat. Aluminiumsilikat kommt bekanntlich als Gemisch von Aluminiumoxid (Al₂O₃) und Silika (SiO₂) vor, wobei das Mengenverhältnis Al₂O₃:SiO₂ über einen breiten Bereich variieren kann. Der Aluminiumsilikat-Katalysator, welcher sich zur Katalyse der hier beschriebenen Dealkoxylierung sehr gut eignet, weist zweckmässigerweise eine mittlere spezifische Oberfläche auf, und zwar vorzugsweise eine im Bereich von etwa 5 bis etwa 50 m²/g. Darüber hinaus befindet sich der Aluminiumoxid-Anteil des Aluminiumsilikat-Katalysators vorzugsweise in der α-Form.

Das obige neue Verfahren zur katalytischen Dealkoxylierung eines 1,1,3-Trialkoxy-2-methyl-butans der oben angegebenen und erläuterten allgemeinen Formel VI zum entsprechenden 1-Alkoxy-2-methyl-1,3-butadien der ebenfalls oben angegebenen und erläuterten allgemeinen Formel II in der Gasphase ist Gegenstand einer Teilanmeldung zur vorliegenden Anmeldung und ist dadurch gekennzeichnet ist, dass man als Katalysator Aluminiumsilikat mittlerer spezifischer Oberfläche verwendet.

Unter Verwendung eines solchen Katalysators, vorzugsweise eines mit einer spezifischen Oberfläche im Bereich von etwa 5 bis etwa 50 m²/g und darüber hinaus eines, dessen Aluminiumoxid-Anteil in der α-Form vorliegt, kann erfahrungsgemäss eine Abspaltungsselektivität von etwa 90% bei einem Umsatz von etwa 90% erreicht werden.

Das neue Verfahren wird zweckmässigerweise analog den bekannten Dealkoxylierungen in der Gasphase durchgeführt, wobei das kennzeichnende Merkmal der neuen Dealkoxylierung in der Verwendung des Aluminiumsilikat-Katalysators besteht. Es gelten für dieses Dealkoxylierungsverfahren zweckmässigerweise Reaktionstemperaturen im Bereich von etwa 250°C bis etwa 400°C, vorzugsweise Temperaturen von etwa 300°C bis etwa 350°C, und zweckmässigerweise Drücke von etwa 1 kPa bis etwa 200 kPa. Vorzugsweise wird das Dealkoxylierungsverfahren bei Atmosphärendruck durchgeführt.

Zweckmässigerweise erfolgt die Dealkoxylierung in einem Festbettreaktor, wobei das Edukt 1,1,3-Trialkoxy-2-methyl-butan, gegebenenfalls mit einem Inertgas verdünnt, kontinuierlich über den Aluminiumsilikat-Katalysator als Festbett in einer Reaktionssäule geleitet wird und das resultierende entsprechende 1-Alkoxy-2-methyl-1,3-butadien am Ende der Reaktionssäule entnommen wird. Die Methodik ist an sich bekannt, was ebenfalls für die dazu benötigten Apparaturen gilt. Als Inertgas kann beispielsweise Stickstoff oder Kohlendioxid verwendet werden. Die Verweilzeiten am Katalysatorbett liegen zweckmässigerweise im Bereich von etwa 0,1 bis etwa 10 Sekunden, vorzugsweise im Bereich von etwa 0,5 bis etwa 5 Sekunden. Durch Einstellung der Reaktionstemperatur, Verweilzeit und weiterer Reaktionsparameter kann der Umsatz an Edukt optimiert werden, damit eine möglichst hohe Ausbeute an gewünschtem Produkt erzielt und möglichst wenig Nebenprodukte gebildet werden.

Wie eingangs erwähnt, kann der erfindungsgemäss hergestellte γ-Chlor- oder γ-Bromtiglinaldehyd der Formel I zur Herstellung von verschiedenen weiteren Zwischenprodukten verwendet werden, welche ihrerseits schliesslich zur Herstellung von Apocarotinalen, Diapocarotinalen sowie Vitamin A von Nutzen sind.

So kann der γ-Halogentiglinaldehyd beispielsweise zur Herstellung des γ-Acetoxy-tiglinaldehyds, eines wichtigen Bausteins für die Herstellung von Vitamin A, eingesetzt werden [siehe Angew. Chem. 72, 811 ff. (1960)]. Er kann ausgehend vom γ-Halogentiglinaldehyd auf vielen Wegen hergestellt werden [siehe u.a. Angew. Chem.100, 301 ff. (1988), DOS 1.227.000, 2.844.949, 2.943.407 und 3.639.562, Bull. Soc. Chim. France 1955, 209 ff., J.Gen. Chem. USSR 32, 1088 ff. (1962; englische Uebersetzung), Russian Chem. Rev. 38, 237 ff. (1969) und Pure & Appl. Chem. 47, 173 ff. (1976)].

Ein attraktiver Weg von γ-Halogentiglinaldehyd zum γ-Acetoxy-tiglinaldehyd besteht darin, das erstere Tiglinaldehydderivat oder ein Acetal davon, insbesondere eines der Formel III, mit Natrium- oder Kaliumacetat umzusetzen. So wurde gemäss J. Gen. Chem. USSR 32, 1088 ff. (1962) durch Umsetzung des γ-Bromtiglinaldehyd-diethylacetals mit Kaliumacetat in rückflussierendem Ethanol 1,1-Diethoxy-4-acetoxy-2-methyl-2-buten in etwa 78%iger Gewichtsausbeute (allerdings ohne Reinheitsangabe) erhalten. Ebenfalls bekannt ist, dass der γ-Halogentiglinaldehyd mit Natrium- oder Kaliumacetat in Wasser bei etwa 100°C in guter Ausbeute (84-86%) zum γ-Acetoxy-tiglinaldehyd umgesetzt werden kann, und zwar aus der DOS 1.227.000. Zusätzlich zu diesen bekannten Methoden wurde nun gefunden, dass die Substitution mit Natrium- oder Kaliumacetat ebenfalls sehr leicht wasserfrei und mit hoher Ausbeute in einem organischen Lösungsmittel, z.B. Toluol, und in Gegenwart eines Phasentransferkatalysators, z.B. Tetraethylammoniumbromid, durchgeführt werden kann. Die Durchführung dieser Substitution unter wasserfreien Bedingungen steht in starkem Gegensatz zur Lehre der DOS 1.227.000, in welcher behauptet wird, dass bei der Umsetzung von γ-Chlor- oder γ-Bromtiglinaldehyd mit Metallsalzen von Carbonsäuren, z.B. mit Kaliumacetat, unter wasserfreien Bedingungen keine zufriedenstellenden Substitutionsausbeuten erzielt werden konnten. Die neue Variante erlaubt eine einfache Aufarbeitung der Reaktion und Isolierung des γ-Acetoxy-tiglinaldehyds, und zwar im letzteren Fall durch Abfiltrieren des gebildeten Alkalihalogenids und des Ueberschusses an Alkaliacetat, gefolgt von einer Destillation des Produktes. Unter Verwendung von Kaliumacetat als Reagens erfolgt die Umsetzung zweckmässigersweise bei etwa 80°C, von dem etwas weniger reaktiven Natriumacetat hingegen zweckmässigerweise bei etwa 110°C. Erwartungsgemäss ist auch der γ-Chlortiglinaldehyd weniger reaktiv als der entsprechende Bromaldehyd.

Die neue Verfahrensvariante kann zudem als Fortsetzung eines "Durchprozesses" erfolgen, und zwar ausgehend von einem 1-Alkoxy-2-methyl-1,3-butadien der Formel II über ein γ-Halogentiglinaldehyd-dialkylacetal der Formel III und den entsprechenden γ-Halogentiglinaldehyd der Formel I, d.h. als Nachstufe des oben definierten erfindungsgemässen Verfahrens.

Als Alternative zur Ueberführung des erfindungsgemäss hergestellten γ-Chlor- oder γ-Bromtiglinaldehyds in den γ-Acetoxy-tiglinaldehyd kann der γ-Halogentiglinaldehyd durch Umsetzung mit Triphenylphosphin in das entsprechende, bekannte C₅-Wittigaldehydhalogenid (nachfolgend "Wittigaldehydsalz") der Formel

Hal⁻Ph₃P⁺CH₂-CH=C(CH₃)-CHO VII

worin Hal Chlor oder Brom und Ph Phenyl bedeuten,
übergeführt werden. Dieses Wittigaldehydsalz bzw. dessen geschützte, insbesondere acetalisierte, Form ist bekanntlich ein wichtiger Baustein für die Herstellung verschiedener Apocarotinale, z.B. β-Apo-8'-carotinal (siehe DOS 1.210.780) und Diapocarotinale, z.B. Crocetindialdehyd (siehe Finn. Chem. Lett. 1984, 102 ff.). Es kann gemäss der Fachliteratur aus dem γ-Chlor- oder γ-Bromtiglinaldehyd durch Umsetzung mit Triphenylphosphin in Toluol bei etwa 110°C (siehe Belgische Patentschrift 656.433) oder in Diethylether [Helv. Chim. Acta 54, 1369 ff. (1971)] leicht hergestellt werden. Die Herstellung des C₅-Wittigaldehydbromids ist auch in Finn. Chem. Lett. 1984, 102 ff. beschrieben. Zusätzlich zu diesen bekannten Methoden wurde nun gefunden, dass die Umsetzung von γ-Chlortiglinaldehyd mit Triphenylphosphin in einem Lösungsmittelgemisch aus Toluol und Acetonitril bei etwa 80°C nach etwa fünfstündiger Reaktionszeit das Wittigaldehydsalz in etwa 67%iger Ausbeute und in hoher Reinheit (grösser als 99%) ergibt. Es ist im allgemeinen festgestellt worden, dass sich das C₅-Wittigaldehydbromid unter milderen Reaktionsbedingungen als das entsprechende Chlorid aus dem diesbezüglichen γ-Halogentiglinaldehyd und Triphenylphosphin herstellen lässt, und zwar bereits bei etwa 40°C in Toluol oder einem Gemisch, vorzugsweise 1:1-Gemisch, aus Toluol und Acetonitril in etwa 2 Stunden gegenüber bei 80°C im erwähnten Lösungsmittelgemisch über 5-8 Stunden. Andere Lösungsmittel, wie beispielsweise Ethylacetat oder Cyclohexan - jeweils allein verwendet - können bei der Bildung des Wittigaldehydsalzes verwendet werden, wobei allerdings erfahrungsgemäss die Ausbeuten etwas niedriger sind.

Es hat sich als praktisch erwiesen, bei der Aufarbeitung der vorhergehenden Chlor- oder Bromalkoxylierung und Hydrolyse dasselbe Lösungsmittel, z.B. Toluol, zum Extrahieren zu verwenden, welches nachher zur Wittigaldehydsalzbildung benötigt wird. Auf diese Weise kann das Wittigaldehydsalz vorteilhaft im Rahmen eines Durchprozesses II → III → I → VII hergestellt werden, zumal der sonst zu isolierende und eventuell auch noch zu reinigende γ-Chlor- oder γ-Bromtiglinaldehyd der Formel I nicht besonders stabil ist.

Die Erfindung wird anhand der nachfolgenden Beispiele veranschaulicht, wobei nicht nur das erfindungsgemässe Verfahren, sondern auch die Herstellung der Ausgangsmaterialien sowie die Verwendung der Endprodukte exemplifiziert sind.

### A. Herstellung des 1-Alkoxy-2-methyl-1,3-butadiens (Verbindung der Formel II)

### Beispiel 1

### 1-Methoxy-2-methvl-1.3-butadien [zwei Stufen a) und b)]

### a) 1.1.3-Trimethoxy-2-methyl-butan

In einem mit Magnetrührer, Thermometer und Tropftrichter ausgestatteten 2 l-Zweihalsrundkolben wurden unter Argon 1035 g (11 Mol) Acetaldehyd-dimethylacetal [Reinheit nach Gaschromatographie (GC): 96,5%] und 3,4 g (3 ml, 24 mMol, 0,22 Mol%) Bortrifluoridetherat vorgelegt. Zum Gemisch wurden innert 2 Stunden bei zwischen 30 und 40°C unter gelegentlichem Kühlen (die Reaktion war exotherm) 530 g (7,35 Mol) Methyl(1-propenyl)-ether zugetropft. Nach beendeter Zugabe wurde noch 30 Minuten bei 30°C weitergerührt, dann auf Raumtemperatur abgekühlt. Man gab 3 ml Triethylamin zu, rührte 15 Minuten und nutschte ab. Der Ueberschuss an Acetaldehyd-dimethylacetal wurde nun an einer Raschig-Kolonne (50 x 3 cm) bei Normaldruck abdestilliert, und der Rückstand an der gleichen Kolonne fraktioniert. Dies ergab bei einem Druck von 50 mbar eine 1. Fraktion (Sdp. 81,5-83,5°C), bestehend aus 742 g (60%ige Ausbeute) 1,1,3-Trimethoxy-2-methyl-butan (Gehalt nach GC: 96,4%) als farblose Flüssigkeit, und eine 2. Fraktion (Sdp. 83,5-85°C), bestehend aus 87,5 g (6,3%ige Ausbeute) 1,1,3-Trimethoxy-2-methyl-butan (Gehalt nach GC: 86%), ebenfalls als farblose Flüssigkeit, d.h. eine totale Ausbeute von 66,3% des gewünschten Produktes.

### b) 1-Methoxy-2-methyl-1,3-butadien

### (i) (1. Variante)

Als Reaktor wurde ein elektrisch geheiztes Stahlrohr (Länge 60 cm, Durchmesser 2,7 cm, Wandtemperatur 300°C) verwendet. Das Reaktionsrohr war unten mit Keramikkugeln (10 cm hoch, Durchmesser 6 mm) gefüllt. Darüber befanden sich 100 ml Aluminiumsilikat-Katalysatorträger mit einer spezifischen Oberfläche von 15 m²/g. Der obere Teil des Reaktors wurde mit Keramikkugeln aufgefüllt. Dem Reaktor wurden kontinuierlich 60 ml/h 1,1,3-Trimethoxy-2-methyl-butan und 20 l/h Stickstoff zugeführt. Die Reaktionsprodukte wurden in einem auf 20°C gekühlten Kondensator mit eingebauter Fritte (Nebelabscheidung) aus dem Stickstoffstrom kondensiert, flüssig entnommen und gaschromatographisch analysiert. Der Umsatz an 1,1,3-Trimethoxy-2-methyl-butan betrug 88%, die Selektivität zu 1-Methoxy-2-methyl-1,3-butadien 90%. Nach Abtrennung des gebildeten Methanols durch Extraktion mit alkalischem Wasser (pH >8) wurde die organische Phase durch Rektifikation aufgetrennt. Man erhielt 1-Methoxy-2-methyl-1,3-butadien mit einer Reinheit von etwa 99%.

### (ii) (2. Variante)

Die Wandtemperatur des Reaktors wurde auf 250°C eingestellt. Alle anderen Bedingungen waren gleich wie in Beispiel b)(i). Der Umsatz an 1,1,3-Trimethoxy-2-methyl-butan betrug 41%, die Selektivität zu 1-Methoxy-2-methyl-1,3-butadien 90%.

### (iii) (3. Variante)

Die Wandtemperatur des Reaktors wurde auf 325°C eingestellt. Alle anderen Bedingungen waren gleich wie in Beispiel b)(i). Der Umsatz an 1,1,3-Trimethoxy-2-methyl-butan betrug 97,5%, die Selektivität zu 1-Methoxy-2-methyl-1,3-butadien 86%.

### Beispiel 2

### 1-Ethoxy-2-methyl-1,3-butadien [zwei Stufen a) und b)]

### a) 1,1,3-Triethoxy-2-methyl-butan

In einem mit Magnetrüher, Thermometer und Tropftrichter ausgestatteten 2 l-Zweihalsrundkolben wurden unter Argon 900 g (7,4 Mol) Acetaldehyd-diethylacetal (Reinheit nach GC: 97%) und 0,85 g (0,75 ml, 6 mMol, 0,25Mol%) Bortrifluoridetherat vorgelegt. Unter gelegentlicher Eiskühlung wurden bei einer Temperatur von etwa 35°C, höchstens aber bei 40°C, 220 g (2,5 Mol) Ethyl-(1-propenyl)-ether innert etwa 30 Minuten zugetropft. Nach beendeter Zugabe wurde noch 30 Minuten bei Raumtemperatur nachgerührt. Dann gab man 4 g festes, pulverisiertes Natriumcarbonat zu und rührte 2,5 Stunden bei Raumtemperatur nach. Dann wurde abgenutscht und der Ueberschuss an Acetaldehyd-diethylacetal an einer Raschig-Kolonne (30 x 2,5 cm) bei 100 mbar abdestilliert. Bei einem Siedepunkt von 40-43°C wurde auf diese Weise etwa 11 Acetaldehyd-diethylacetal (Gehalt nach GC: 91%) zurückerhalten. Der Rückstand wurde nun bei einem Druck von 12-13 mbar an der gleichen Kolonne fraktioniert. Dies ergab 344,5 g (66,2%ige Ausbeute) 1,1,3-Triethoxy-2-methyl-butan als wasserklare Flüssigkeit mit einem Siedepunkt von 81-84°C und einem Gehalt nach GC von 98,2%.

### b) 1-Ethoxy-2-methyl-1,3-butadien

Dem im Beispiel 1b) beschriebenen Reaktor (gleiche Temperatur, gleicher Katalysator) wurden 60 ml/h 1,1,3-Triethoxy-2-methyl-butan und 20 l/h Stickstoff zugeführt. Vom eingesetzten 1,1,3-Triethoxy-2-methyl-butan wurden 90% umgesetzt; die Selektivität zu 1-Ethoxy-2-methyl-1,3-butadien betrug etwa 90%. Die Reaktionsprodukte wurden analog zu Beispiel 1 aufgearbeitet. Man erhielt 1-Ethoxy-2-methyl-1,3-butadien mit einer Reinheit von etwa 98%.

### B. Herstellung des γ-Halogentiglinaldehyds (Verbindung der Formel I)

### Beispiel 3

### γ-Chlortiglinaldehyd

In einem 350 ml-Vierhalssulfierkolben wurden 10 g (82 mMol) 1-Ethoxy-2-methyl-1,3-butadien in 100 ml Ethanol gelöst. Die Lösung wurde dann auf 0°C gekühlt und innert einer Stunde tropfenweise mit 90 g (160 mMol) 13%iger wässriger Natriumhypochloritlösung versetzt, was einen weissen Niederschlag ergab. Dann wurde etwa 30 Minuten bei Raumtemperatur nachgerührt, auf Wasser gegossen und viermal mit 100 ml, total 400 ml, n-Pentan extrahiert. Die gesamte Pentanlösung wurde etwa eine Stunde bei Raumtemperatur mit 250 ml 10%iger Schwefelsäure gerührt. Anschliessend wurde die Wasserphase abgetrennt und die organische Phase der Reihe nach mit 100 ml Natriumbicarbonatlösung und 100 ml Natriumchloridlösung gewaschen, über wasserfreiem Natriumsulfat getrocknet und unter vermindertem Druck eingeengt. Dies ergab 7 g einer gelben Flüssigkeit, welche man an 200 g Kieselgel (0,04-0,063 mm) mit Cyclohexan/ Ethylacetat (9:1) chromatographierte. Man erhielt nach Kugelrohrdestillation bei etwa 100°C/15 mm Hg 3,5 g (35%ige Ausbeute) γ-Chlortiglinaldehyd als farblose Flüssigkeit mit einer Reinheit nach GC von 97,1%.

| | | | | |
|---|---|---|---|---|
| Mikroanalyse: | Ber. | C 50,65% | H 5,95% | Cl 29,90% |
| | Gef. | C 50,59% | H 6,09% | Cl 29,78% |

¹H-NMR (250 MHz, CDCl₃): 1,83 ppm (s, 3H), 4,30 ppm (d, J ≅ 8 Hz, 1H), 6,57 ppm (t, J ∼ 8 Hz, 1H), 9,49 ppm (s, 1H); IR (Film): 1692, 1648; Massenspektrum: 118 (M⁺).

### Beispiel 4

### γ-Chlortiglinaldehyd

In einem mit mechanischem Rührer, Tropftrichter und Thermometer ausgestatteten 500 ml-Vierhalssulfierkolben wurden unter Argon 22,7 g (0,2 Mol) 1-Ethoxy-2-methyl-1,3-butadien (Reinheit nach GC: 99%) in 200 ml Methanol vorgelegt. Bei etwa +5°C wurden innert 15 Minuten (wegen exothermer Reaktion) 140 g (0,24 Mol, 1,2 Aeq.) 13%ige wässrige Natriumhypochloritlösung unter Rühren zugetropft. Nach beendeter Zugabe wurde noch 15 Minuten bei +5°C weitergerührt. Dann wurden nochmals 10 g (0,02 Mol, 0,1 Aeq.) 13%ige wässrige Natriumhypochloritlösung bei 0°C zugetropft. Das Reaktionsgemisch wurde nun auf 150 ml Wasser gegossen, zweimal mit je 50 ml, total 100 ml, n-Pentan extrahiert. Die wässrige Phase wurde nun mit Natriumchlorid gesättigt und nochmals mit 50 ml n-Pentan extrahiert.

Die gesamte organische Phase wurde nun mit 25 ml Wasser gewaschen und die zweiphasige Lösung etwa 30 Minuten bei Raumtemperatur mit 50 ml 6N-Salzsäure gerührt. Die wässrige Phase wurde nun abgetrennt, mit Natriumchlorid gesättigt und mit 50 ml n-Pentan extrahiert. Danach wurde die vereinigte organische Phase mit 25 ml gesättigter Natriumbicarbonatlösung gewaschen, über wasserfreiem Natriumsulfat getrocknet, filtriert und unter vermindertem Druck vorsichtig eingeengt. Dies ergab 23 g rohen γ-Chlortiglinaldehyd als hellgelbe Flüssigkeit mit einer Reinheit nach GC von etwa 75%. Das Rohprodukt wurde nicht spektroskopisch untersucht, sondern unmittelbar gemäss Beispiel 13 mit Triphenylphosphin umgesetzt.

### Beispiel 5

### γ-Chlortiglinaldehyd

Analog der in Beispiel 4 beschriebenen Arbeitsweise wurden 20,5 g (0,2 Mol) 1-Methoxy-2-methyl-1,3-butadien (Reinheit nach GC: 96%) in 200 ml Methanol bei +5°C mit insgesamt 150 g (0,26 Mol, 1,3 Aeq.) 13%iger wässriger Natriumhypochloritlösung umgesetzt. Anschliessende analoge Hydrolyse (100 ml 3N Salzsäure/Raumtemperatur/etwa 30 Minuten) und Aufarbeitung ergab 22 g rohen γ-Chlortiglinaldehyd als gelbliches Oel mit einer Reinheit nach GC von etwa 77%. Auch dieses Rohprodukt wurde nicht spektroskopisch untersucht, sondern unmittelbar gemäss Beispiel 14 mit Triphenylphosphin umgesetzt.

### Beispiel 6

### γ-Chlortiglinaldehyd

Ebenfalls analog der in Beispiel 4 beschriebenen Arbeitsweise wurden 22,7 g (0,2 Mol) 1-Ethoxy-2-methyl-1,3-butadien (Reinheit nach GC: 99%) in 200 ml Methanol bei 0°C mit 150 g (etwa 0,26 Mol, 1,3 Aeq.) 13%iger wässriger Natriumhypochloritlösung 30 Minuten umgesetzt. Anschliessende analoge Aufarbeitung unter Verwendung von 150 ml n-Pentan gefolgt von saurer Hydrolyse des Acetals mit 50 ml 6N Salzsäure bei 0°C über 30 Minuten ergab 26,8 g rohen γ-Chlortiglinaldehyd mit einer Reinheit nach GC von etwa 73% als dunkelgelbes Oel. Das Rohprodukt wurde nicht spektroskopisch untersucht, sondern unmittelbar gemäss Beispiel 15 in γ-Acetoxy-tiglinaldehyd überführt.

### Beispiel 7

### γ-Chlortiglinaldehyd

In einem mit Magnetrührer, Tropftrichter und Thermometer ausgestatteten 200 ml-Vierhalssulfierkolben wurden 10,1 g (0,1 Mol) 1-Methoxy-2-methyl-1,3-butadien in 100 ml Methanol gelöst. Die Lösung wurde dann auf -20°C gekühlt und innert 30 Minuten tropfenweise mit 12,0 g (0,11 Mol, 1,1 Aeq.) tert.Butylhypochlorit versetzt. [Für die Herstellung dieses Hypochlorits siehe Vogel's Textbook of Practical Organic Chemistry, 5. Bd., Longman Group U.K. Ltd., 1989, Seite 422; Ed. B.S. Furniss et al.].

Die erhaltene klare Lösung wurde auf 200 ml Wasser gegossen und dreimal mit je 50 ml, total 150 ml, n-Pentan extrahiert. Die vereinigten Pentanphasen wurde der Reihe nach mit 25 ml 5%iger Natriumbisulfitlösung und mit 25 ml Wasser gewaschen, dann etwa 30 Minuten bei 0°C mit 25 ml 6N-Salzsäure versetzt und gerührt. Nach üblicher Aufarbeitung wurden 12,1 g roher γ-Chlortiglinaldehyd als gelbliche Flüssigkeit erhalten. Das Rohprodukt wurde nicht spektroskopisch untersucht, sondern unmittelbar gemäss Beispiel 16 mit Triphenylphosphin umgesetzt.

### Beispiel 8

### γ-Bromtiglinaldehyd

In einem mit Magnetrührer und Thermometer ausgestatteten 200 ml-Vierhalssulfierkolben wurden 10,1 g (0,1 Mol) 1-Methoxy-2-methyl-1,3-butadien in 50 ml Methanol bei etwa -7°C bis -12°C unter Rühren portionenweise innert 30 Minuten mit 15,6 g (0,11 Mol) N-Bromacetamid versetzt. Das Reaktionsgemisch wurde dann 30 Minuten bei -10°C nachgerührt. Das Gemisch wurde nun zu 100 ml 2,5%iger Natriumbicarbonatlösung gegeben und das Ganze dreimal mit je 50 ml, total 150 ml, n-Pentan extrahiert.

Man wusch die vereinigten organischen Phasen mit 25 ml Wasser, versetzte sie anschliessend unter Argon mit 30 ml 6N Salzsäure und rührte das zweiphasige Gemisch bei 0°C eine Stunde. Die wässrige Phase wurde nun abgetrennt, und zweimal mit je 25 ml, total 50 ml, n-Pentan extrahiert. Danach wurden die vereinigten organischen Phasen mit 25 ml gesättigter Natriumbicarbonatlösung gewaschen, über wasserfreiem Natriumsulfat getrocknet, filtriert und unter vermindertem Druck eingeengt. Dies ergab 15,5 g rohen γ-Bromtiglinaldehyd als gelbe Flüssigkeit. Das Rohprodukt wurde nicht spektroskopisch untersucht, sondern unmittelbar gemäss Beispiel 17 mit Triphenylphosphin umgesetzt.

### Beispiel 9

### γ-Chlortiglinaldehyd

In einem mit Magnetrührer und Thermometer ausgestatteten 200 ml-Vierhalssulfierkolben wurden unter Argon 10,1 g (0,1 Mol) 1-Methoxy-2-methyl-1,3-butadien (Reinheit nach GC: 97,5%) mit 50 ml Methanol vorgelegt. Unter Rühren wurden dann bei etwa -10°C bis -5°C 12 g (98%ig rein, 0,06 Mol) 1,3-Dichlor-5,5-dimethyl-hydantoin zugegeben, und zwar portionenweise innert etwa 30 Minuten. Nach beendeter Zugabe wurde noch etwa eine Stunde bei 0°C weitergerührt. Das Gemisch, welches aus einer farblosen Flüssigkeit und einem beigefarbenen Niederschlag bestand, wurde nun auf 200 ml destilliertes Wasser gegossen und dreimal mit je 50 ml, total 150 ml, n-Pentan extrahiert.

Die vereinigten organischen Phasen wurden mit 25 ml Wasser gewaschen und die zweiphasige Lösung etwa eine Stunde bei 0°C mit 25 ml 6N Salzsäure gerührt. Man trennte die wässrige Phase dann ab und extrahierte sie zweimal mit je 25 ml, total 50 ml, n-Pentan. Schliesslich wurde die gesamte organische Phase mit 25 ml gesättigter Natriumbicarbonatlösung gewaschen, über wasserfreiem Natriumsulfat getrocknet, filtriert und unter vermindertem Druck eingeengt. Dies ergab 11,9 g rohen γ-Chlortiglinaldehyd als gelbliche Flüssigkeit mit einer Reinheit nach GC von etwa 77%. Das Rohprodukt wurde nicht spektroskopisch untersucht, sondern unmittelbar gemäss Beispiel 18 mit Triphenylphosphin umgesetzt.

### Beispiel 10

### γ-Bromtiglinaldehyd

In einem mit Magnetrührer und Thermometer ausgestatteten 200 ml-Vierhalssulfierkolben wurden unter Argon 10,1 g (0,1 Mol) 1-Methoxy-2-methyl-1,3-butadien (Reinheit nach GC: 97,5%) mit 50 ml Methanol vorgelegt. Unter Rühren wurden dann bei etwa -15°C bis -10°C 16 g (97%ig rein, 0,054 Mol) 1,3-Dibrom-5,5-dimethyl-hydantoin zugegeben, und zwar portionenweise innert etwa 30 Minuten. Nach beendeter Zugabe wurde noch etwa 30 Minuten bei -10°C weitergerührt. Das Gemisch, welches aus einer gelblichen Flüssigkeit und einem weissen Niederschlag bestand, wurde nun auf 100 ml gesättigte Natriumbicarbonatlösung gegossen und dreimal mit je 50 ml, total 150 ml, n-Pentan extrahiert.

Die vereinigten organischen Phasen wurden mit 25 ml destilliertem Wasser gewaschen und die zweiphasige Lösung etwa eine Stunde bei 0°C mit 30 ml 6N Salzsäure gerührt. Man trennte die wässrige Phase dann ab und extrahierte sie zweimal mit je 25 ml, total 50 ml, n-Pentan. Schliesslich wurde die gesamte organische Phase mit 25 ml gesättigter Natriumbicarbonatlösung gewaschen, über wasserfreiem Natriumsulfat getrocknet, filtriert und unter vermindertem Druck eingeengt. Dies ergab 16,6 g rohen γ-Bromtiglinaldehyd als gelbliche Flüssigkeit mit einer Reinheit nach GC von etwa 95%. Das Rohprodukt wurde nicht spektroskopisch untersucht, sondern unmittelbar gemäss Beispiel 19 mit Triphenylphosphin umgesetzt.

### Beispiel 11

### γ-Bromtiglinaldehyd (und dessen Ueberführung in das C₅-Wittigaldehydbromid der Formel VII in Rahmen eines Durchprozesses)

In einem mit mechanischem Rührer, Tropftrichter und Thermometer ausgestatteten 350 ml-Vierhalssulfierkolben wurden 11,7 g (0,1 Mol) 1-Ethoxy-2-methyl-1,3-butadien (Reinheit nach GC: 96%) in 100 ml Methanol vorgelegt. Bei -10°C wurden unter starkem Rühren 65 g (0,125 Mol) durch Lösen von 11 g (0,28 Mol) Natriumhydroxid-Plätzchen in 35 ml Wasser und Zutropfen von etwa 6,6 ml (20,5 g, 0,13 Mol) Brom bei 0°C in etwa 30 Minuten (pH etwa 12,7-12,9) frisch hergestellte 23%ige Natriumhypobromit-Lösung innert 45 Minuten zugetropft und dann noch 15 Minuten bei -10°C nachgerührt. Dann wurde das Reaktionsgemisch auf 50 ml Toluol gegossen und mit 100 ml Wasser gewaschen. Die wässrige, milchige Wasserphase wurde nochmals mit 50 ml Toluol extrahiert. Die gesamte organische Phase wurde nun mit 25 ml Wasser gewaschen und die resultierende zweiphasige Lösung anschliessend unter Argon mit 30 ml 6N wässrige Salzsäure bei 0°C 45 Minuten magnetisch gerührt. Die wässrige Phase wurde abgetrennt und nochmals mit 25 ml Toluol extrahiert. Die vereinigten Toluolphasen wurden nun mit 25 ml gesättigter Natriumbicarbonatlösung gewaschen, über wasserfreiem Natriumsulfat getrocknet, filtriert und in einen mit mechanischem Rührer und Thermometer ausgestatteten 350 ml-Vierhalssulfierkolben unter Argon gegeben.

Zur Lösung wurden unter Rühren 23,5 g (0,09 Mol, 0,9 Aeq.) Triphenylphosphin gegeben, und das Gemisch wurde etwa 16 Stunden bei 40°C gerührt (Kristallisation begann schon nach einigen Minuten bei Raumtemperatur). Die resultierende beige Suspension wurde nun auf 0°C innert 15 Minuten abgekühlt, abgenutscht und dreimal mit je 25 ml, total 75 ml, Toluol bei 0°C gewaschen. Nach Trocknen wurden 30,0 g (69,8%) C₅-Wittigaldehyd-bromid als weisses, kristallines Pulver mit Smp. 256-257°C und einem Gehalt nach HPLC von 98,95% erhalten.

### Beispiel 12

### γ-Bromtiglinaldehyd (und dessen Ueberführung in den γ-Acetoxy-tiglinaldehyd in Rahmen eines Durchprozesses)

Analog der in Beispiel 11 beschriebenen Arbeitsweise wurden 11,7 g (0,1 Mol) 1-Ethoxy-2-methyl-1,3-butadien in 100 Methanol bei -10°C mit 70 g (etwa 0,135 Mol) 23%iger Natriumhypobromit-Lösung umgesetzt. Analoge dreimalige Extraktion mit je 50 ml, total 150 ml, Toluol, Hydrolyse mit 30 ml 6N Salzsäure bei 0°C sowie Extraktion der salzsauren Phase mit zusätzlichen 50 ml Toluol ergab eine Lösung des γ-Bromtiglinaldehyds in etwa 200 ml Toluol.

Diese Lösung wurde nach Trocknen über wasserfreiem Natriumsulfat in einem mit mechanischem Rührer, Kühler und Thermometer ausgestattetten 350 ml -Vierhalssulfierkolben unter Argon mit 15 g (0,15 Mol) Kaliumacetat und 1 g (4,8 Mol, 5 Mol%) Tetraethylammoniumbromid bei 80°C 2 1/2 Stunden gerührt. Das Gemisch wurde nun auf +10°C abgekühlt, filtriert und unter vermindertem Druck vorsichtig eingeengt. Dies ergab 18,6 g rohen γ-Acetoxy-tiglinaldehyd als gelbliche Flüssigkeit. Destillation (Vigreux) bei 20 mbar ergab bei einem Siedepunkt von 101-103°C 8,4 g (56,4%ige Ausbeute) γ-Acetoxy-tiglinaldehyd als gelbe Flüssigkeit mit einem Gehalt nach GC von 95,4%.

### C. Ueberführung des γ-Halogentiglinaldehyds der Formel I in das C₅-Wittigaldehyd-halogenid bzw. den γ-Acetoxy-tiglinaldehyd.

### Beispiel 13

### C₅-Wittigaldehyd-Chlorid

Das Rohprodukt des Beispiels 4 wurde in einen mit Magnetrührer und Kühler ausgestatteten 500 ml-Rundkolben in 80 ml Acetonitril und 80 ml Toluol gelöst. Man versetzte dann die Lösung mit 57 g (0,22 Mol) Triphenylphosphin und rückflussierte etwa 5 Stunden bei etwa 85°C. Die resultierende Suspension (eine gelb-orange Lösung mit beigem Niederschlag) wurde auf etwa +3°C abgekühlt, abgenutscht und zweimal mit je 50 ml, total 100 ml, Toluol gewaschen. Das Filtergut wurde nun 3 Stunden bei 50°C unter Wasserstrahlvakuum getrocknet. Dies ergab 51,0 g (66,6 %ige Ausbeute bezogen auf 1-Ethoxy-2-methyl-1,3-butadien) C₅-Wittigaldehyd-Chlorid als leicht beiges kristallines Pulver mit einem Schmelzpunkt von 247-248°C und einem Gehalt nach HPLC von 99,4%.

| | | | | |
|---|---|---|---|---|
| Mikroanalyse: | Ber. | C 72,53% | H 5,82% | Cl 9,31% |
| | Gef. | C 72,23% | H, 5,75% | Cl 9,40% |

### Beispiel 14

### C₅-Wittigaldehyd-Chlorid

Analog der in Beispiel 13 beschriebenen Arbeitsweise wurde das Rohprodukt des Beispiels 5 mit 53 g (0,2 Mol) Triphenylphosphin in 80 ml Acetonitril und 80 ml Toluol umgesetzt. Dies ergab 51,0 g (66,5 %ige Ausbeute bezogen auf 1-Methoxy-2-methyl-1,3-butadien) C₅-Wittigaldehyd-Chlorid als weisses Pulver mit einem Schmelzpunkt von 247-248°C und einem Gehalt nach HPLC von 99,2%.

### Beispiel 15

### γ-Acetoxy-tiglinaldehyd

Das Rohprodukt des Beispiels 6 wurde in einem mit mechanischem Rührer ausgestatteten Vierhalssulfierkolben in 200 ml Toluol aufgenommen und mit 25 g (0,25 Mol, 1,25 Aeq.) Kaliumacetat und 600 mg (2,8 mMol, 1,5 Mol%) Tetraethylammoniumbromid versetzt. Das Gemisch wurde bei Rückflusstemperatur 1 1/2 Stunden unter Rühren gekocht. Dann wurde die Suspension auf 5°C abgekühlt, filtriert und unter vermindertem Druck vorsichtig eingedampft. Dies ergab 18,8 g rohen γ-Acetoxytiglinaldehyd als orangebraunes Oel. Eine Destillation an einer (20 cm/ Ø 14,5) Vigreux-Kolonne bei 11 mbar ergab als Hauptfraktion (Sdp. 90-92,5°C) 14,9 g (46%ige Ausbeute bezogen auf 1-Ethoxy-2-methyl-1,3-butadien) γ-Acetoxy-tiglinaldehyd als gelbes Oel mit einem Gehalt nach GC von 87,8%.

### Beispiel 16

### C₅-Wittigaldehyd-Chlorid

Analog der in Beispiel 13 beschriebenen Arbeitsweise wurde das Rohprodukt des Beispiels 7 mit 26,2 g (0,1 Mol) Triphenylphosphin in 40 ml Acetonitril und 40 ml Toluol umgesetzt, und zwar 18 Stunden bei Rückflusstemperatur. Dies ergab 28,0 g (73,4%ige Ausbeute bezogen auf 1-Methoxy-2-methyl-1,3-butadien) C₅-Wittigaldehyd-Chlorid als weisses Pulver mit einem Schmelzpunkt von 246-247°C und einem Gehalt nach HPLC von 99,7%.

### Beispiel 17

### C₅-Wittigaldehyd-Bromid

Analog der in Beispiel 13 beschriebenen Arbeitsweise wurde das Rohprodukt des Beispiels 8 mit 27,5 g (0,105 Mol) Triphenylphosphin in 50 ml Acetonitril und 50 ml Toluol umgesetzt, und zwar 2 Stunden bei 40°C. Dies ergab 34,1 g (80,1%ige Ausbeute bezogen auf 1-Methoxy-2-methyl-1,3-butadien) C₅-Wittigaldehyd-Bromid als schneeweisses, kristallines Pulver mit einem Schmelzpunkt von 254-255°C und einem Gehalt nach HPLC von 98,7%.

### Beispiel 18

### C₅-Wittigaldehyd-Chlorid

Analog der in Beispiel 13 beschriebenen Arbeitsweise wurde das Rohprodukt des Beispiels 9 mit 26,2 g (0,1 Mol) Triphenylphosphin in 40 ml Acetonitril und 40 ml Toluol umgesetzt, und zwar 16 Stunden bei Rückflusstemperatur. Dies ergab 27,6 g (72,5%ige Ausbeute bezogen auf 1-Methoxy-2-methyl-1,3-butadien) C₅-Wittigaldehyd-Chlorid als weisses Pulver mit einem Schmelzpunkt von 247-248°C.

### Beispiel 19

### C₅-Wittigaldehyd-Bromid

Analog der in Beispiel 13 beschriebenen Arbeitsweise wurde das Rohprodukt des Beispiels 10 mit 27,5 g (etwa 1,05 Aeq.) Triphenylphosphin in 50 ml Acetonitril und 50 ml Toluol umgesetzt, und zwar 2 Stunden bei 40°C und anschliessend weitere etwa 45 Minuten bei 0°C. Dies ergab 36,4 g (85,6%ige Ausbeute bezogen auf 1-Methoxy-2-methyl-1,3-butadien) C₅-Wittigaldehyd-Bromid als schneeweisses Pulver mit einem Schmelzpunkt von 255-256°C (Zersetzung).

## Patentansprüche

1. Verfahren zur Herstellung von γ-Chlor- oder γ-Bromtiglinaldehyd der allgemeinen Formel
HalH₂C-CH=C(CH₃)-CHO I
worin Hal Chlor oder Brom bedeutet,
**dadurch gekennzeichnet, dass** man ein 1-Alkoxy-2-methyl-1,3-butadien der allgemeinen Formel
H₂C=CH-C(CH₃)=CH-OR¹ II
worin R¹ C₁₋₄-Alkyl bedeutet,
mittels eines Halogenierungsmittels, welches aus einem Alkalimetallhypochlorit, einem Alkalimetallhypobromit, einem Erdalkalimetallhypochlorit, einem Erdalkalimetallhypobromit, tert.Butylhypochlorit, N-Bromacetamid, 1,3-Dichlor-5,5-dimethyl-hydantoin und 1,3-Dibrom-5,5-dimethyl-hydantoin ausgewählt ist, in einem Alkohol R²OH, worin R² C₁₋₄-Alkyl bedeutet, haloalkoxyliert, und das so erhaltene γ-Halogentiglinaldehyd-dialkylacetal der allgemeinen Formel
HalH₂C-CH=C(CH₃)-CH(OR¹)(OR²) III
worin Hal, R¹ und R² die oben angegebenen Bedeutungen besitzen,
zum gewünschten γ-Chlor- bzw. γ-Bromtiglinaldehyd der Formel I hydrolysiert.

2. Verfahren nach Anspruch 1, **dadurch gekennzeichnet, dass** R¹ und R² beide Methyl oder Ethyl bedeuten.

3. Verfahren nach Anspruch 1 oder 2, **dadurch gekennzeichnet, dass** als Halogenierungsmittel ein Alkalimetall- oder Erdalkalimetallhypochlorit bzw. -hypobromit, vorzugsweise Natriumhypochlorit, Kaliumhypochlorit, Natriumhypobromit oder Kaliumhypobromit, verwendet wird.

4. Verfahren nach Anspruch 3, **dadurch gekennzeichnet, dass** zur Haloalkoxylierung eine wässrige Lösung des Alkalimetallhypochlorits bzw. -hypobromits verwendet wird, die eine Konzentration an Hypohalogenit zwischen 5% w/w und 30% w/w, vorzugsweise zwischen 10% w/w und 20% w/w, aufweist.

5. Verfahren nach einem der Ansprüche 1 bis 4, **dadurch gekennzeichnet, dass** pro Aequivalent 1-Alkoxy-2-methyl-1,3-butadien der Formel II zwischen 1,1 und 1,4 Aequivalente des ein Halogenatom aufweisenden Halogenierungsmittels oder zwischen 0,55 und 0,7 Aequivalente Erdalkalimetallhypochlorit oder -hypobromit oder 1,3-Dichlor- oder 1,3-Dibrom-5,5-dimethyl-hydantoin, sowie ein zwischen zehnfach und dreissigfach liegender Ueberschuss des Alkohols R²OH eingesetzt werden.

6. Verfahren nach einem der Ansprüche 1 bis 5, **dadurch gekennzeichnet, dass** die Haloalkoxylierung im Temperaturbereich von -20°C bis zur Raumtemperatur, vorzugsweise bei Temperaturen zwischen -10°C und +5°C, erfolgt.

7. Verfahren nach einem der Ansprüche 1 bis 6, **dadurch gekennzeichnet, dass** man zur Hydrolyse das nach beendeter Haloalkoxylierung erhaltene Gemisch mit Wasser vereinigt, das daraus resultierende wässrige Gemisch mit einem organischen Lösungsmittel extrahiert und die so erhaltene Lösung des γ-Halogentiglinaldehyd-dialkylacetals der Formel III im organischen Lösungsmittel mit einer wässrigen Mineralsäure versetzt.

8. Verfahren nach Anspruch 7, **dadurch gekennzeichnet, dass** als organisches Lösungsmittel ein aliphatischer, alicyclischer oder aromatischer Kohlenwasserstoff, ein halogenierter aliphatischer Kohlenwasserstoff, ein aliphatischer Ether oder ein aliphatischer Ester, vorzugsweise n-Pentan, n-Hexan, Toluol, Methylenchlorid, Chloroform, Diethylether, tert.Butylmethylether bzw. Ethylacetat, und als Mineralsäure Schwefelsäure oder Salzsäure verwendet wird.

## Claims

1. A process for the manufacture of γ-chloro- or γ-bromotiglic aldehyde of the general formula
HalH₂C-CH=C(CH₃)-CHO I
wherein Hal signifies chlorine or bromine,
which process comprises haloalkoxylating a 1-alkoxy-2-methyl-1,3-butadiene of the general formula
H₂C=CH-C(CH₃)=CH-OR¹ II
wherein R¹ signifies C₁₋₄-alkyl,
using a halogenating agent selected from an alkali metal hypochlorite, an alkali metal hypobromite, an alkaline earth metal hypochlorite, an alkaline earth metal hypobromite, tert.butyl hypochlorite, N-bromoacetamide, 1,3-dichloro-5,5-dimethyl-hydantoin and 1,3-dibromo-5,5-dimethyl-hydantoin in an alcohol R²OH, wherein R² signifies C₁₋₄-alkyl, and hydrolyzing the thus-obtained γ-halotiglic aldehyde dialkyl acetal of the general formula
HalH₂C-CH=C(CH₃)-CH(OR¹)(OR²) III
wherein Hal, R¹ and R² have the significances given above,
to the desired γ-chloro- or γ-bromotiglic aldehyde of formula I.

2. A process according to claim 1, wherein R¹ and R² both signify methyl or ethyl.

3. A process according to claim 1 or 2, wherein an alkali metal or alkaline earth metal hypochlorite or hypobromite, preferably sodium hypochlorite, potassium hypochlorite, sodium hypobromite or potassium hypobromite, is used as the halogenating agent.

4. A process according to claim 3, wherein an aqueous solution of the alkali metal hypochlorite or hypobromite which has a hypohalite concentration between 5% w/w and 30% w/w, preferably between 10% w/w and 20% w/w, is used for the haloalkoxylation.

5. A process according to any one of claims 1-4, wherein per equivalent of 1-alkoxy-2-methyl-1,3-butadiene of formula II there are used between 1.1 and 1.4 equivalents of the halogenating agent having one halogen atom or between 0.55 and 0.7 equivalent of alkaline earth metal hypochlorite or hypobromite or 1,3-dichloro- or 1,3-dibromo-5,5-dimethylhydantoin as well as an excess of the alcohol R²OH which is between ten fold and thirty fold.

6. A process according to any one of claims 1-5, wherein the haloalkoxylation is effected in a temperature range of -20°C to room temperature, preferably at temperatures between -10°C and +5°C.

7. A process according to any one of claims 1-6, wherein, for the hydrolysis, the mixture obtained after completion of the haloalkoxylation is combined with water, the aqueous mixture resulting therefrom is extracted with an organic solvent and the thus-obtained solution of the γ-halotiglic aldehyde dialkyl acetal of formula III in the organic solvent is treated with an aqueous mineral acid.

8. A process according to claim 7, wherein an aliphatic, alicyclic or aromatic hydrocarbon, a halogenated aliphatic hydrocarbon, an aliphatic ether or an aliphatic ester, preferably n-pentane, n-hexane, toluene, methylene chloride, chloroform, diethyl ether, tert.butyl methyl ether or ethyl acetate, is used as the organic solvent and sulphuric acid or hydrochloric acid is used as the mineral acid.

## Revendications

1. Procédé pour la préparation du γ-chloro- ou γ-bromotiglinaldéhyde de formule générale
HalH₂C-CH=C(CH₃)-CHO I
dans laquelle Hal représente le chlore ou le brome,
**caractérisé en ce qu'**on soumet à une halogéno-alcoxylation un 1-alcoxy-2-méthyl-1,3-butadiène de formule générale
H₂C=CH-C(CH₃)=CH-OR¹ II
dans laquelle R¹ représente un groupe alkyle en C₁₋₄,
au moyen d'un agent d'halogénation qui est choisi parmi un hypochlorite de métal alcalin, un hypobromite de métal alcalin, un hypochlorite de métal alcalino-terreux, un hypobromite de métal alcalino-terreux, l'hypochlorite de tert-butyle, le N-bromo-acétamide, la 1,3-dichloro-5,5-diméthylhydantoïne et la 1,3-dibromo-5,5-diméthylhydantoïne, dans un alcool R²OH, dans lequel R² représente un groupe alkyle en C₁₋₄, et on soumet à une hydrolyse le γ-halogénotiglinaldéhyde-dialkylacétal ainsi obtenu, de formule générale
HalH₂C-CH=C(CH₃)-CH(OR¹)(OR²) III
dans laquelle Hal, R¹ et R² ont les significations données ci-dessus,
pour aboutir au γ-chloro- ou γ-bromotiglinaldéhyde recherché de formule I.

2. Procédé selon la revendication 1, **caractérisé en ce que** R¹ et R² représentent l'un et l'autre le groupe méthyle ou éthyle.

3. Procédé selon la revendication 1 ou 2, **caractérisé en ce qu'**on utilise comme agent d'halogénation un hypochlorite ou hypobromite de métal alcalin ou de métal alcalino-terreux, de préférence l'hypochlorite de sodium, l'hypochlorite de potassium, l'hypobromite de sodium ou l'hypobromite de potassium.

4. Procédé selon la revendication 3, **caractérisé en ce qu'**on utilise pour l'halogéno-alcoxylation une solution aqueuse de l'hypochlorite ou hypobromite de métal alcalin qui présente une concentration d'hypohalogénite comprise entre 5 % p/p et 30 % p/p, de préférence entre 10 % p/p et 20 % p/p.

5. Procédé selon l'une quelconque des revendications 1 à 4, **caractérisé en ce qu'**on utilise, par équivalent de 1-alcoxy-2-méthyl-1,3-butadiène de formule II, entre 1,1 et 1,4 équivalent de l'agent d'halogénation comportant un atome d'halogène ou entre 0,55 et 0,7 équivalent d'hypochlorite ou d'hypobromite de métal alcalino-terreux ou de 1,3-dichloro- ou 1,3-dibromo-5,5-diméthylhydantoïne, ainsi qu'un excès se situant entre 10 et 30 fois de l'alcool R²OH.

6. Procédé selon l'une quelconque des revendications 1 à 5, **caractérisé en ce qu'**on effectue l'halogéno-alcoxylation dans la plage de températures allant de -20°C à la température ambiante, de préférence à des températures comprises entre -10°C et +5°C.

7. Procédé selon l'une quelconque des revendications 1 à 6, **caractérisé en ce que**, pour l'hydrolyse, on unit avec de l'eau le mélange obtenu une fois terminée l'halogéno-alcoxylation, on extrait le mélange aqueux résultant avec un solvant organique et on ajoute un acide minéral aqueux à la solution ainsi obtenue du γ-halogénotiglinaldéhyde-dialkylacétal de formule III dans le solvant organique.

8. Procédé selon la revendication 7, **caractérisé en ce qu'**on utilise comme solvant organique un hydrocarbure aliphatique, alicyclique ou aromatique, un hydrocarbure aliphatique halogéné, un éther aliphatique ou un ester aliphatique, de préférence le n-pentane, le n-hexane, le toluène, le chlorure de méthylène, le chloroforme, l'oxyde d'éthyle, l'éther tert-butylméthylique ou l'acétate d'éthyle, et, comme acide minéral, l'acide sulfurique ou l'acide chlorhydrique.
